# EUROPEAN PATENT APPLICATION

(11) **EP 3 646 887 A1**
(43) Date of publication of application: **06.05.2020**
(21) Application number: 18823953.7
(22) Date of filing: 02.07.2018
(51) Int. Cl.: A61K 47/64, C07K 14/71, C07K 16/00, A61K 39/395, A23L 33/10, A23K 20/10

(54) **CONJUGATE OF VEGF-GRAB PROTEIN AND DRUG, AND USE THEREOF**

(30) Priority: 30.06.2017 KR 20170083747
(71) Applicant: Korea Advanced Institute of Science and Technology, Daejeon 34141 (KR)
(72) Inventor: KIM, Ho Min, Daejeon 34141 (KR); LEE, Dae Hee, Daejeon 34141 (KR); KIM, Duk Ki, Jongno-gu, Seoul 03128 (KR)
(74) Representative: Mewburn Ellis LLP
(86) International application number: PCT/KR2018/007465
(87) International publication number: WO 2019/004799

(57) **Abstract**

The present invention relates to a VEGF-Grab protein-drug conjugate and a use thereof and, more particularly, to a conjugate of a fusion protein in which a VEGFR1 domain 2, a VEGFR1 domain 3, and an antibody fragment are connected and a drug, a pharmaceutical composition for prevention or treatment of cancer or angiogenesis-related disease, comprising the conjugate, and a method for prevention or treatment of cancer or angiogenesis-related disease. Serving as a multi-paratopic VEGF decoy receptor, the conjugate including a VEGF-Grab protein and a drug of the present invention can be used as a multi-purpose platform for treatment of cancer or angiogenesis-related disease.

## Description

### [Technical Field]

The present invention relates to a conjugate of a VEGF-Grab protein and a drug, and a use thereof, more particularly to a conjugate of a fusion protein, in which a VEGFR1 domain 2, a VEGFR1 domain 3, an antibody fragment are linked to one another and a drug, a pharmaceutical composition for the prevention or treatment of cancer or angiogenesis-related disease which comprises the conjugate, and a method of preventing or treating cancer or angiogenesis-related disease.

### [Background Art]

For the proliferation and growth of cancer cells, new blood vessels supplying oxygen and nutrients are needed, and a vascular endothelial growth factor (VEGF) is known to play a pivotal role in the formation of new blood vessels. VEGF is a dimer of about 46 kDa consisting of two subunits, and five types of VEGFs (VEGF-A, VEGF-B, VEGF-C, VEGF-D, and PlGF) are currently known in mammals. VEGF binds to three receptor tyrosine kinases (RTKs) known as VEGF receptors (VEGFR)-1, - 2, and -3, and these VEGF receptors cause cell migration, survival, proliferation, and the like and have functions of transmitting a signal capable of forming three-dimensional blood vessels, which are not present in other RTKs, or regulating vascular permeability.

The expression of VEGF molecules is increased in tumor cells, and the number of VEGF receptors is increased in tumor-infiltrating vascular endothelial cells, but since both VEGF and VEGF receptors were found to be expressed at low levels in normal cells which are not related to angiogenesis, VEGF has been used as a target for cancer treatment.

Accordingly, new anti-cancer therapies for blocking the production of blood vessels that supply nutrients to cancer cells, rather than cancer cells themselves, have been developed, and it has been reported that anti-VEGF receptor antibodies, soluble decoy receptor structures, antisense, RNA aptamers for VEGF, low-molecular-weight VEGF receptor tyrosine kinase (RTK) inhibitors, and the like can be used to interfere with VEGF signaling. Anti-VEGF neutralizing antibodies have been found to inhibit the growth of various human tumor cell lines in nude mice (Warren et al. J. Clin. Invest. 95: 1789-1797 (1995)). In addition, various VEGF inhibitors are disclosed in patent documents related to VEGF inhibitors, such as quinazoline derivatives as VEGF inhibitors (US Patent No. 9040548), inhibitors of VEGF receptors and HGF receptor signaling for treating angiogenesis-mediated cell proliferative diseases or inhibiting solid tumor growth (US Patent No. 8470850), and an angiogenesis-inhibiting substance used for the treatment of diseases such as cancer and the like by hindering the binding between VEGF and receptor thereof (Korean Patent No. 2003-0075947).

However, conventional angiogenesis inhibitors are useful in treating cancer because they inhibit angiogenesis needed for cancer cell proliferation, but such inhibitors do not have a function of targeting tumor cells, and thus could not exhibit cancer cell-specific anti-cancer efficacy and caused a harmful effect on normal blood vessels. In the case of Bevacizumab (Trade Name: Avastin™), commercialized as a humanized antibody against VEGF-A, side effects such as excessive intestinal hemorrhage, hemoptysis, cerebral hemorrhage, nasal bleeding, and hematemesis upon coughing were observed in phase III clinical trials performed by Genentech, and observation of headaches, elevated blood pressure, nasal edema, proteinuria, dry skin, excessive tears, back pain, skin edema, and the like has also been reported. These side effects may be regarded as being caused because conventional angiogenesis inhibitors have no function of targeting tumor cells.

### [Disclosure]

### [Technical Problem]

Under these backgrounds, the present inventors have made extensive efforts to develop an angiogenesis inhibitor which is effectively delivered to cancer cells by selectively targeting cancer cells, as a result, the present inventors have confirmed that a fusion protein-drug conjugate according to the present invention is able to efficiently and selectively inhibit angiogenesis of cancer cells and to not only inhibit cancer growth but also minimize side effects induced by anti-cancer agents, thereby completing the present invention.

### [Technical Solution]

It is an object of the present invention to provide a conjugate of a fusion protein and a drug, wherein the fusion protein in which a VEGFR1 domain 2, a VEGFR1 domain 3, and an antibody fragment are linked to one another.

It is another object of the present invention to provide a pharmaceutical composition for the prevention or treatment of cancer or angiogenesis-related disease comprising the conjugate.

It is a further object of the present invention to provide a method of preventing or treating cancer or angiogenesis-related disease, comprising administering the conjugate to a subject.

It is a further object of the present invention to provide a polynucleotide encoding the conjugate.

It is a further object of the present invention to provide an expression vector comprising the polynucleotide.

It is a further object of the present invention to provide a transformant comprising the expression vector.

It is a further object of the present invention to provide a method of producing a conjugate comprising culturing the transformant.

It is a further object of the present invention to provide a conjugate of a fusion protein and a drug for use in prevention or treatment of cancer or angiogenesis-related disease, wherein the fusion protein in which a VEGFR1 domain 2, a VEGFR1 domain 3, and an antibody fragment that are linked to one another.

It is a further object of the present invention to provide a use of a conjugate of a fusion protein and a drug for the manufacture of a medicine for preventing or treating cancer or angiogenesis-related disease, wherein the fusion protein in which a VEGFR1 domain 2, a VEGFR1 domain 3, and an antibody fragment that are linked to one another.

### [Advantageous Effects]

A conjugate comprising a VEGF-Grab protein and a drug, according to the present invention, is a multi-paratopic VEGF decoy receptor, and can be used as a multipurpose platform for treating cancer or angiogenesis-related diseases.

### [Description of Drawings]

FIG. 1 illustrates the structures and SDS-PAGE analysis results of Cet-Grab and Tras-Grab.
FIG. 2 illustrates the binding affinities of Cet-Grab and Tras-Grab.
FIG. 3 illustrates the effects of inhibiting vascular endothelial cell migration and tube formation through inhibition of the VEGF signaling pathways by Cet-Grab and Tras-Grab.
FIG. 4 illustrates the effect of inducing cancer cell death through blocking of EGFR pathway-mediated cell proliferation signaling by Cet-Grab and Tras-Grab.
FIG. 5 illustrates the results of colony formation analysis to investigate anti-tumor effect by Cet-Grab and Tras-Grab.
FIG. 6 illustrates tumor-specific targeting results of Cet-Grab and Tras-Grab in xenograft mouse models.
FIG. 7 illustrates a Cet-Grab treatment scheme and the effect of Cet-Grab on inhibiting tumor growth in EGFR+ A431 xenograft mouse models.
FIG. 8 illustrates the effect of Cet-Grab on inhibiting EGFR signaling.
FIG. 9 illustrates the effect of Cet-Grab on inhibiting angiogenesis and changes in concentrations of VEGF-A and P1GF.
FIG. 10 illustrates a Tras-Grab treatment scheme in HER2+ SKOV3 xenograft mouse models.
FIG. 11 illustrates the effect of Tras-Grab on inhibiting tumor growth.

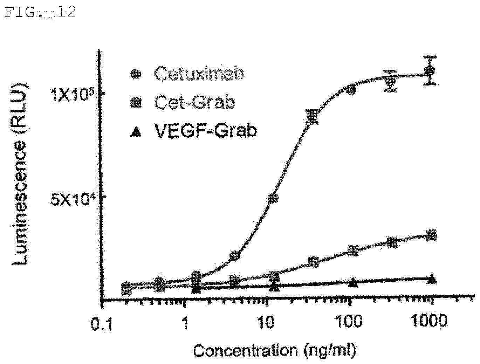

FIG. 12 illustrates concentration-dependent cytotoxicity of Cet-Grab.

### [Best Mode For Carrying Out The Invention]

A conjugate according to the present invention is a conjugate in which a fusion protein including a VEGFR1 domain 2, a VEGFR1 domain 3, an Fc antibody fragment, and a drug are bound to each other, and the conjugate may bind to cancer cells targeted by the drug, inhibit the phosphorylation of VEGFR-2 by binding to VEGF, and selectively suppress angiogenesis in the vicinity of cancer cells by preventing the differentiation of vascular endothelial cells, thereby inhibiting cancer growth.

Hereinafter, the present invention will be described in more detail.

Meanwhile, each description and embodiment disclosed in the present invention may also be applied to other descriptions and embodiments. In other words, all combinations of the various elements disclosed in the present invention fall within the scope of the present invention. In addition, the specific descriptions provided below are not intended to limit the scope of the present application. In addition, one of ordinary skill in the art may recognize or identify numerous equivalents to specific embodiments described herein using only general experiments. In addition, these equivalents are intended to fall within the scope of the present invention.

To achieve the above objects, an embodiment of the present invention provides a conjugate of a fusion protein and a drug, wherein the fusion protein comprising a VEGFR1 domain 2, a VEGFR1 domain 3, and an antibody fragment.

The term "vascular endothelial growth factor receptor 1 (VEGFR1)" as used herein refers to a receptor of vascular endothelial growth factor (VEGF), and VEGFR1 may stimulate cell division, migration, differentiation, and the like by activating the tyrosine kinase of the receptor. In addition, VEGFR1 domains 2 and 3 are domains that recognize VEGF. Since there is a difference in amino acid sequences of proteins exhibiting activity depending on species, the VEGFR1 domains 2 and 3 are not limited in terms of the origins or sequences thereof, and may include wild types thereof or variants thereof having activity. The VEGFR1 domain 2 according to the present invention may comprise the amino acid sequence of SEQ ID NO: 27 or an amino acid sequence encoded by the nucleotide sequence of SEQ ID NO: 28, and the VEGFR1 domain 3 may comprise the amino acid sequence of SEQ ID NO: 29 or an amino acid sequence encoded by the nucleotide sequence of SEQ ID NO: 30.

The term "antibody fragment" as used herein means any portion of an antibody, and the antibody fragment is divided into a fragment antigen-binding (Fab) region, which is an antigen-binding site, and a fragment crystallizable (Fc) region, which is a region that does not bind to an antigen.

In addition, the term "antibody Fc region" as used herein refers to heavy chain constant region 2 (CH2) and heavy chain constant region 3 (CH3), except for heavy chain and light chain variable regions, heavy chain constant region 1 (CH1), and light chain constant region (CL1) of immunoglobulin, and the antibody Fc region may include a hinge portion in the heavy chain constant region. The antibody Fc region is a biodegradable polypeptide which is metabolized *in vivo,* and thus is safely used as a carrier for drugs. In addition, the immunoglobulin Fc region is advantageous in terms of preparation, purification, and yield of conjugates because of its relatively low molecular weight compared to the whole immunoglobulin molecule, and since amino acid sequences thereof are different according to antibody, the effects of greatly increasing the homogeneity of a substance and reducing the likelihood of inducing blood antigenicity may be expected by removing Fab moieties, which show high heterogeneity.

The Fc region of an antibody may be an Fc region derived from IgG, IgA, IgD, IgE, or IgM, or a combination or hybrid thereof, particularly derived from IgG or IgM which is most abundant in human blood, and more particularly derived from human-derived IgG1, but the present invention is not limited thereto.

The term "fusion protein" as used herein refers to an artificially synthesized protein in which a VEGFR1 domain 2, a VEGFR1 domain 3, and an antibody fragment are bound, and particularly, the fusion protein may include the VEGFR1 domain 2, the VEGFR1 domain 3, and the antibody fragment. In addition, the fusion protein may be formed such that a VEGFR1 domain 2, a VEGFR1 domain 3, and an antibody fragment, or a VEGFR1 domain 3, a VEGFR1 domain 2, and an antibody fragment are linked from the N terminus in that order. In the fusion protein, the VEGFR1 domain 2, the VEGFR1 domain 3, and the antibody fragment may be directly linked to each other or may also be linked via a linker.

The linker is not particularly limited as long as it allows the fusion protein to exhibit activity, but particularly includes an amino acid such as glycine, alanine, leucine, isoleucine, proline, serine, threonine, asparagine, aspartic acid, cysteine, glutamine, glutamic acid, lysine, arginine acid, and the like, more particularly several amino acids selected from valine, leucine, aspartic acid, glycine, alanine, proline, and the like, and more particularly 1 to 20 amino acids selected from glycine, valine, leucine, aspartic acid, and the like, in consideration of the ease of genetic manipulation.

In addition, in the present invention, the fusion protein may be in a form in which a VEGFR1 domain 2, a VEGFR1 domain 3, and an antibody fragment are linked to one another, and the antibody fragment may comprise an Fc region of an antibody, may particularly be a protein including VEGFR1 domain 2(R1D2)-VEGFR1 domain 3(R1D3)-hinge-Fc regions (CH2 and CH3) of a human antibody, and may be used interchangeably with the term VEGF-Grab. In addition, the VEGF-Grab may comprise the known VEGFR1 domain 2 and VEGFR1 domain 3, and, as a non-limiting example, water-soluble decoy receptor VEGF-Grab (Lee JE, Mol Cancer Ther. 2015, 14:470-9.) or VEGF-Trap (Holash J, Proc Natl Acad Sci. USA 2002, 99:11393-8.) may be used.

The fusion protein binds to VEGF, which is a ligand of vascular endothelial growth factor receptor 1 (VEGFR1), and particularly binds to VEGF-A, VEGF-B, or PlGF to inhibit the activity thereof. The fusion protein according to the present invention may comprise the amino acid sequence of SEQ ID NO: 22, or may comprise an amino acid sequence encoded by the nucleotide sequence of SEQ ID NO: 23.

The fusion protein may comprise a polypeptide having a sequence in which at least one amino acid residue is different from the amino acid sequence of a wild type of each domain included therein. Amino acid exchanges in proteins and polypeptides that do not alter the overall activity of molecules are known in the art. The most commonly occurring exchanges are exchanges between amino acid residues Ala/Ser, Val/Ile, Asp/Glu, Thr/Ser, Ala/Gly, Ala/Thr, Ser/Asn, Ala/Val, Ser/Gly, Thy/Phe, Ala/Pro, Lys/Arg, Asp/Asn, Leu/Ile, Leu/Val, Ala/Glu, and Asp/Gly. In addition, the fusion protein may comprise a protein having enhanced structural stability to withstand heat, pH, or the like or enhanced activity, due to variation or modification of the amino acid sequence.

The fusion protein or a polypeptide consisting the fusion protein may be prepared through a chemical peptide synthesis method known in the art, or may be prepared by amplifying a gene encoding the fusion protein through a polymerase chain reaction (PCR) or synthesizing the gene using a known method, cloning the gene into an expression vector, and expressing the gene.

In the present invention, the drug may be an anti-cancer agent or a therapeutic agent for angiogenesis-related disease.

The term "anti-cancer agent" as used herein refers collectively to drugs used in chemotherapy for cancer treatment, and the term "cancer" refers to an abnormally grown tumor attributable to autonomous overgrowth of body tissues or a tumor-forming disease.

The term "angiogenesis" as used herein refers to a physiological process in which new blood vessels are produced, and may be used interchangeably with the term "neovascularization" as used herein. In addition, "angiogenesis-related disease" refers to a disease caused by excessive angiogenesis, and examples thereof include tumor growth and metastasis, diabetic retinopathy, premature retinopathy, corneal graft rejection, neovascular glaucoma, erythrosis, proliferative retinopathy, psoriasis, macular degeneration, hemophiliac joints, capillary proliferation within atherosclerotic plaques, keloid, wound granulation, vascular adhesion, rheumatoid arthritis, chronic inflammation, osteoarthritis, autoimmune disease, Crohn's disease, restenosis, atherosclerosis, intestinal stenosis, cat scratch disease, ulcers, cirrhosis complications, glomerulonephritis, diabetic nephropathy, malignant nephrosclerosis, thrombotic micro vascular syndrome, organ transplant rejection, glomerulopathy, diabetes, inflammation or neurodegeneration.

In the present invention, the drug may be an antibody capable of binding to human epidermal growth factor receptor type2 (HER2) or epidermal growth factor receptor (EGFR), particularly trastuzumab or cetuximab, but is not limited thereto.

In addition, in the present invention, the drug may be an antibody having a form selected from the group consisting of scFv, dsFv, Fab, Fab', F(ab')₂ and nanobody, which are antigen recognition sites, particularly an antibody having a scFv form, but is not limited thereto.

The term "antigen recognition site" as used herein refers to any fragment of an antibody of the present invention that retains the antigen-binding activity of an antibody, and is used interchangeably with "antigen-binding fragment" and "binding fragment of a peptide".

The Fab has the variable regions of a light chain and a heavy chain, the constant regions of the light chain, and the first constant region (CH1 domain) of the heavy chain, and has one antigen-binding site. Fab' is different from Fab in that the Fab' has the hinge region including at least one cysteine residue at the C-terminus of the heavy chain CH1 domain. A F(ab')₂ antibody is produced when cysteine residues of the hinge region of the Fab' form a disulfide bond. A variable fragment (Fv) refers to the minimal antibody fragment having only the heavy chain variable region and the light chain variable region. Two-chain disulfide Fv (dsFv) has a structure in which the heavy chain variable region is linked to the light chain variable region by a disulfide bond, and single-chain Fv (scFv) generally has a structure in which the heavy chain variable region is covalently bound to the light chain variable regions via a peptide linker. These antibody fragments may be obtained using proteolytic enzymes (e.g., a whole antibody can be digested with papain to obtain Fab, or can be restriction-digested with pepsin to obtain F(ab')₂ fragments), and preferably may be prepared by a genetic recombinant technique. In addition, the nanobody is an antibody-derived therapeutic protein having the unique structure and functional properties of naturally occurring heavy-chain antibodies, wherein the heavy-chain antibodies include a single variable domain (VH) and two constant domains (CH2 and CH3).

In the present invention, the anti-cancer agent may be trastuzumab or cetuximab.

The term "trastuzumab" as used herein refers to an antibody capable of specifically binding to cancer cells, and means an anti-HER2 monoclonal antibody. Trastuzumab specifically binds to cancer cells by recognizing cancer-related antigens which are specifically expressed or excessively expressed on cancer cell surfaces or tissues, particularly the HER2 protein, but are not limited thereto. The term "trastuzumab" may be used interchangeably with the trade name Herceptin™.

The drug included in the conjugate according to the present invention may be a single-chain variable fragment (scFv) of trastuzumab. The scFv of trastuzumab may comprise the amino acid sequence of SEQ ID NO: 20, or may comprise an amino acid sequence encoded by the nucleotide sequence of SEQ ID NO: 21. Specifically, the scFv of trastuzumab may be in a form in which a heavy chain variable region of trastuzumab comprising the amino acid sequence of SEQ ID NO: 14 and a light chain variable region of trastuzumab comprising the amino acid sequence of SEQ ID NO: 16 are linked to each other via a linker comprising the amino acid sequence of SEQ ID NO: 18, or may be in a form in which a heavy chain variable region of trastuzumab comprising the amino acid sequence encoded by the nucleotide sequence of SEQ ID NO: 15 and a light chain variable region of trastuzumab comprising the amino acid sequence encoded by the nucleotide sequence of SEQ ID NO: 17 are linked to each other via a linker comprising an amino acid sequence encoded by the nucleotide sequence of SEQ ID NO: 19, but the present invention is not limited thereto.

In addition, the scFv of trastuzumab may bind to a HER2 receptor. The term "human epidermal growth factor receptor type2 (HER2)" as used herein refers to an epidermal growth factor receptor (EGFR) family, and HER2 is the most potent oncoprotein in breast cancer. Normal expression of HER2 is involved in the growth and development of mammary tissues, but abnormal overexpression or amplification of HER2 leads to broken balance of regulation, resulting in the formation of aggressive cancer cells in mammary tissues. The scFv of trastuzumab binds to HER2 overexpressed in cancer cells.

In addition, the term "cetuximab" as used herein refers to an antibody capable of specifically binding to cancer cells, and means an anti-EGFR monoclonal antibody. Cetuximab specifically binds to cancer cells by recognizing a cancer-related antigen, particularly the EGFR protein, which is specifically expressed or excessively expressed on cancer cell surfaces or tissues, but is not limited thereto. The term "cetuximab" can be used interchangeably with the trade name Erbitux™.

The drug included in the conjugate according to the present invention may be a single-chain variable fragment (scFv) of cetuximab. The scFv of cetuximab may comprise the amino acid sequence of SEQ ID NO: 9, or may comprise an amino acid sequence encoded by the nucleotide sequence of SEQ ID NO: 10. Specifically, the scFv of cetuximab may be in a form in which a heavy chain variable region of cetuximab comprising the amino acid sequence of SEQ ID NO: 3 and a light chain variable region of cetuximab comprising the amino acid sequence of SEQ ID NO: 5 are linked to each other via a linker comprising the amino acid sequence of SEQ ID NO: 7, or may be in a form in which a heavy chain variable region of cetuximab comprising an amino acid sequence encoded by the nucleotide sequence of SEQ ID NO: 4 and a light chain variable region of cetuximab comprising an amino acid sequence encoded by the nucleotide sequence of SEQ ID NO: 6 are linked to each other via a linker comprising an amino acid sequence encoded by the nucleotide sequence of SEQ ID NO: 8, but the present invention is not limited thereto.

In addition, the scFv of cetuximab may bind to an EGFR receptor. The term "EGFR" refers to a member of the epidermal growth factor receptor (EGFR) family, and abnormal activation of EGFR causes many epithelial cell tumors, including lung cancer. The abnormal activation of EGFR causes continuous cell proliferation, invasion of surrounding tissues, distant metastasis, and angiogenesis, and increases cell survival. The scFv of cetuximab binds to EGFR overexpressed in cancer cells.

The conjugate according to the present invention may be in a form in which the N-terminus of the fusion protein is linked to the C-terminus of the drug, directly or via a linker. Specifically, the conjugate may be in the form of fusion of the C-terminus of an anti-EGFR therapeutic antibody (cetuximab or trastuzumab) scFv to the N-terminus of VEGF-Grab, which are respectively named Cetuximab-VEGF-Grab (Cet-Grab) and Trastuzumab-VEGF-Grab (Tras-Grab). In addition, the term "conjugate" may be used interchangeably with the term "multi-paratopic VEGF decoy receptor".

The term "decoy receptor" as used herein refers to a receptor that is able to recognize and bind to specific growth factors or cytokines efficiently, but is unable to activate a general receptor complex or structurally deliver a signal. The decoy receptor binds to a ligand and prevents the ligand from binding to the receptor, thereby acting as an inhibitor of signaling.

The multi-paratopic VEGF decoy receptors of the present invention, Cetuximab-VEGF-Grab and Trastuzumab-VEGF-Grab, have similar binding affinities with the parent VEGF-Grab and anti-EGFR antibodies (cetuximab and trastuzumab), and thus may simultaneously bind to the VEGF family (VEGF-A and PlGF) and the EGFR family (EGFR for Cet-Grab; and HER2 for Tras-Grab). In addition, it was confirmed that Cetuximab-VEGF-Grab and Trastuzumab-VEGF-Grab effectively inhibited not only VEGF signaling but also signaling of the EGFR family, both *in vitro* and *in vivo,* and particularly enhanced antitumor efficacy in xenograft mouse models compared to VEGF-Grab by acting specifically limited to tumors.

In addition, the conjugate according to the present invention may comprise the amino acid sequence of SEQ ID NO: 11 or SEQ ID NO: 24, or may comprise an amino acid sequence encoded by the nucleotide sequence of SEQ ID NO: 12 or SEQ ID NO: 25.

Another embodiment of the present invention provides a polynucleotide encoding the conjugate.

Here, the definition of the term "conjugate" is the same as given above.

The term "polynucleotide" as used herein refers to a polymer material in which nucleotides are bound, and DNA encoding genetic information.

The sequence of the polynucleotide encoding the conjugate may be easily derived by those of ordinary skill in the art to which the present invention pertains from the amino acid sequence of SEQ ID NO: 11 or 24, and may particularly be the nucleotide sequence of SEQ ID NO: 12 or 25, but the present invention is not limited thereto.

In addition, in the present invention, nucleotide sequences encoding the conjugate, the fusion protein, and drugs comprise not only the nucleotide sequence encoding the amino acid of each SEQ ID NO, but also nucleotide sequences with at least 80% homology, particularly at least 90% homology, more particularly at least 95% homology, still more particularly at least 98% homology, and most particularly at least 99% homology to the above sequence, but are not particularly limited as long as they are nucleotide sequences encoding a protein that exhibits potency substantially the same or equivalent to that of each of the above proteins. In addition, it will be obvious that any amino acid sequence as a sequence having homology to the above sequence, having biological properties substantially the same as or equivalent to those of conjugate proteins with the described SEQ ID NOs, amino acid residues of which are partially deleted, altered, substituted, or inserted, is also within the scope of the present invention.

As used herein, the term "homology" refers to a degree of similarity of nucleotide sequences or amino acid sequences encoding a protein, and when homology is sufficiently high, expression products of the corresponding gene may have the same or similar activity. In addition, homology may be expressed as a percentage depending on the degree of consistency with the given amino acid sequence or nucleotide sequence. In the present specification, homologous sequences thereof having activity the same as or similar to that of the given amino acid sequence or nucleotide sequence are expressed as having "% homology". For example, homology may be confirmed by comparing sequences through a hybridization experiment using standard software for calculating parameters such as score, identity, similarity, and the like, particularly BLAST 2.0, or under defined stringent conditions, and the determination of defined appropriate hybridization conditions is within the scope of the corresponding art, and may be made using a method well known in the art (e.g., J. Sambrook et al., Molecular Cloning, A Laboratory Manual, 2nd Edition, Cold Spring Harbor Laboratory press, Cold Spring Harbor, New York, 1989; F.M. Ausubel et al., Current Protocols in Molecular Biology, John Wiley & Sons, Inc., New York).

The conjugate, the fusion protein, and the drug, according to the present invention, may comprise the amino acid sequence of the corresponding SEQ ID NO. or a polynucleotide encoding a protein with at least 80% homology, at least 85% homology, at least 90% homology, at least 91% homology, at least 92% homology, at least 93% homology, at least 94% homology, at least 95% homology, at least 96% homology, at least 97% homology, at least 98% homology, or at least 99% homology to the above sequence, as long as it has biological activity the same as or equivalent to that of each protein.

In addition, polynucleotides encoding the proteins may have various modifications in an encoding region within a range that does not change the amino acid sequence of the protein expressed by the encoding region, in consideration of codons suitable for use in a living organism to express the protein due to the degeneracy of codons. Therefore, the polynucleotide may comprise any polynucleotide without limitation as long as it is a polynucleotide sequence encoding a corresponding protein.

In addition, probes that can be prepared from known sequences, for example any sequence encoding a protein having the activity of the conjugate, the fusion protein, and the drug through hybridization with sequences complementary to all or part of the polynucleotide sequences under stringent conditions may be included without limitation.

The term "stringent conditions" refers to conditions that enable specific hybridization between polynucleotides. These conditions are specifically set forth in documents (e.g., J. Sambrook et al., same as above). For example, the stringent conditions may include conditions where genes with high homology, at least 40% homology, particularly at least 90% homology, more particularly at least 95% homology, more particularly at least 97% homology, and most particularly at least 99% homology, are hybridized, and genes with homology lower than that are not hybridized, or commonly used washing conditions for hybridization, i.e., washing once, particularly twice or three times at salt concentration and temperature corresponding to 60 °C, 1 X SSC, 0.1% SDS, particularly 60 °C, 0.1 X SSC, 0.1% SDS, and more particularly 68 °C, 0.1 X SSC, 0.1% SDS.

Hybridization requires that two polynucleotides have complementary sequences, although mismatch between bases is possible depending on the stringency of hybridization. The term "complementary" is used to describe the relationship between nucleotide bases that can hybridize with each other. For example, with respect to DNA, adenosine is complementary to thymine and cytosine is complementary to guanine. Thus, the present application may also include isolated polynucleotide fragments that are complementary to the whole sequence as well as substantially similar polynucleotide sequences.

Specifically, polynucleotides having homology may be detected using hybridization conditions including hybridization processes at a Tm value of 55 °C and using the above-described conditions. In addition, the Tm value may be, but is not limited to, 60 °C, 63 °C, or 65 °C, and may be appropriately adjusted by one of ordinary skill in the art according to the purpose of use.

Stringency appropriate for hybridizing polynucleotides depends on the length and degree of complementarity of the polynucleotides, and variables pertinent thereto are well known in the art (see Sambrook et al., Supra, 9.50-9.51, 11.7-11.8) .

Another embodiment of the present invention provides an expression vector comprising the polynucleotide.

Here, the definition of the term "polynucleotide" is the same as provided above.

As used herein, the term "expression vector" refers to a recombinant vector that is introduced into a suitable host cell and can express a target protein, and to a gene construct including essential regulatory elements operably linked to express a gene insert.

The term "operably linked" as used herein means that a regulatory sequence of nucleic acid expression and a nucleic acid sequence encoding a target protein are functionally linked to perform a general function. Operable linkage with a recombinant vector may be performed using genetic recombinant techniques well known in the art, and site-specific DNA cleavage and ligation may be easily performed using enzymes commonly known in the art.

The suitable expression vector of the present invention may include a signal sequence for membrane targeting or secretion in addition to expression control elements such as a promoter, an initiation codon, a termination codon, a polyadenylation signal, and an enhancer. Initiation and termination codons are generally considered to be part of the nucleotide sequence encoding an immunogenic target protein, must have activity in a subject when the gene construct is administered, and must be in frame with an encoding sequence. A general promoter may be constitutive or inducible, and promoters for prokaryotic cells include lac, tac, T3 and T7 promoters, and promoters for eukaryotic cells include a monkey virus 40 (SV40) promoter, a mouse mammary tumor virus (MMTV) promoter, a human immunodeficiency virus (HIV) promoter such as a long terminal repeat (LTR) promoter of HIV, a Moloney virus promoter, a cytomegalovirus (CMV) promoter, an Epstein Barr virus (EBV) promoter, Rous sarcoma virus (RSV) promoters, a β-actin promoter, and promoters derived from human hemoglobin, human muscle creatine, and human metallothionein, but the present invention is not limited thereto.

In addition, the expression vector may include a selective marker for selecting host cells containing the vector. The selective marker functions to select transformed cells using the vector, and markers that impart selectable phenotypes such as drug resistance, auxotrophy, resistance to a cytotoxic agent, or expression of a surface protein may be used. Since only the cells expressing the selective marker survive in an environment treated with a selective agent, the transformed cells may be selected. In addition, in the case where the vector is a replicable expression vector, the vector may include a replication origin, which is a specific nucleic acid sequence in which replication is initiated.

As a recombinant expression vector for inserting a foreign gene, various types of vectors including plasmids, viruses, cosmids, and the like may be used. The type of recombinant vector is not particularly limited, as long as the recombinant vector functions to express a desired gene and produce a desired protein in various types of prokaryote and eukaryote host cells, but particularly, a vector capable of mass-producing a promoter having strong activity and a foreign protein having a shape similar to that of a natural state while retaining strong expression may be used.

To express the fusion protein according to the present invention, various combinations of hosts and vectors may be used. A suitable expression vector for a eukaryotic host cell may include an expression control sequence derived from SV40, bovine papillomatosis, an adenovirus, an adeno-associated virus, a cytomegalovirus, and a retrovirus, but the present invention is not limited thereto. An expression vector usable in a bacterial host includes a bacterial plasmid obtained from *Escherichia coli* such as pET, pRSET, pBluescript, pGEX2T, pUC vector, col E1, pCR1, pBR322, pMB9, or derivatives thereof, a plasmid having a wider host range such as RP4, phage DNA which can be exemplified by λgt10, λgt11, or NM989, and other phage DNA such as M13 and filamentous single-stranded phage DNA, but the present invention is not limited thereto. A 2 °C plasmid or a derivative thereof may be used for yeast cells, and pVL941 or the like may be used for insect cells.

Another embodiment of the present invention provides a transformant comprising the expression vector.

Here, the definition of the term "expression vector" is as given above.

The term "transformant" as used herein may refer to a host cell into which the expression vector can be introduced. Specifically, the transformant of the present invention may be a transformant from a source other than a human, but the present invention is not limited thereto.

The host cell suitable for introduction of the vector may be a prokaryotic host cell such as *E. coli, Bacillus subtilis, Streptomyces* sp., *Pseudomonas* sp., *Proteus mirabilis,* or *Staphylococcus* sp. In addition, the host cell may be fungus such as *Aspergillus* sp., yeast such as *Pichia pastoris, Saccharomyces cerevisiae, Schizosaccharomyces* sp., or *Neurospora crassa,* other lower eukaryotic cells, or higher eukaryotic cell such as plant or insect cells. In addition, the host cell may be a mammalian cell, and specifically, monkey kidney cells (COS7), NSO cells, SP2/0, Chinese hamster ovary (CHO) cells, W138, baby hamster kidney (BHK) cells, MDCK, myeloma cell lines, HuT 78 cells, HEK293 cells, or the like may be used, but the present invention is not limited thereto.

The transformation method of the present invention includes any method of introducing a nucleic acid into an organism, cell, tissue or organ, and may be carried out by selecting a suitable standard technique according to a host cell known in the art. Specifically, the method includes, but is not limited to, electroporation, plasma fusion, calcium phosphate (CaPO₄) precipitation, calcium chloride (CaCl₂) precipitation, agitation using silicon carbide fibers, agrobacterium-mediated transformation, PEG, dextran sulfate, lipofectamine, and dry/inhibition-mediated transformation methods, but the present invention is not limited thereto.

Another embodiment of the present invention provides a method of producing a conjugate comprising culturing the transformant.

Herein, the definition of terms "transformant" and "conjugate" is the same as given above.

The method of producing a conjugate comprises culturing the transformant according to the present invention, and specifically may comprise: constructing an expression vector by inserting a polynucleotide sequence encoding the conjugate into a vector; producing a transformant by introducing the expression vector into a host cell; culturing the transformant; and isolating and purifying a conjugate from the cultured transformant.

More specifically, the conjugate may be mass-produced by culturing the transformant in a nutrition medium, and medium and culture conditions may be appropriately selected and used according to a host cell. Conditions such as temperature, the pH of the medium, the culture time, and the like may be appropriately adjusted to be suitable for cell growth and the mass production of proteins during culture.

The recombinant peptide or protein produced as described above may be collected from the medium or cell lysate. A membrane-binding type may be dissociated from a membrane using a suitable surfactant solution (e.g., Triton-X 100) or by enzymatic cleavage. Cells used in fusion protein expression may be disrupted by various physical or chemical methods such as freeze-thaw purification, sonication, mechanical disruption, or cytolysis, and may be isolated and purified using conventional biochemical separation techniques (Sambrook et al., Molecular Cloning: A Laboratory Manual, 2nd Ed., Cold Spring Harbor Laboratory Press (1989); Deutscher, M., Guide to Protein Purification Methods Enzymology, Vol. 182. Academic Press. Inc., San Diego, CA (1990)). Electrophoresis, centrifugation, gel filtration, precipitation, dialysis, chromatography (ion exchange chromatography, affinity chromatography, immunosorbent chromatography, size exclusion chromatography, and the like), isoelectric focusing, various variations thereof, and various combinations thereof may be used, but the present invention is not limited thereto.

Another embodiment of the present invention provides a pharmaceutical composition for the prevention or treatment of cancer or angiogenesis-related disease, which comprises the conjugate.

Here, the definitions of terms "conjugate", "cancer", and "angiogenesis" are the same as described above.

In the present invention, the cancer is not particularly limited as long as symptoms thereof are alleviated, reduced, improved, and treated by the pharmaceutical composition according to the present invention. In the present invention, the type of cancer is not particularly limited, but may be cancer in which HER2 or EGFR is overexpressed. In addition, the cancer includes both solid and blood cancer, and particularly may be liver cancer, lung cancer, pancreatic cancer, non-small cell lung cancer, colon cancer, bone cancer, skin cancer, head or neck cancer, skin or intraocular melanoma, uterine cancer, ovarian cancer, rectal cancer, gastric cancer, anal muscle cancer, breast cancer, fallopian tube carcinoma, endometrial carcinoma, cervical carcinoma, vaginal carcinoma, vulva carcinoma, Hodgkin's disease, esophageal cancer, small intestine cancer, endocrine adenocarcinoma, thyroid cancer, parathyroid cancer, adrenal cancer, soft-tissue sarcoma, urethral cancer, penile cancer, prostate cancer, chronic or acute leukemia, lymphocytic lymphoma, bladder cancer, kidney or ureter cancer, renal cell carcinoma, renal pelvic carcinoma, central nervous system (CNS) tumor, primary central nervous system lymphoma, spinal cord tumor, brain stem glioma, or pituitary adenoma, and more particularly may be solid cancer, but the present invention is not limited thereto.

In addition, in the present invention, the angiogenesis-related disease is not particularly limited as long as symptoms thereof can be alleviated, reduced, improved, or treated by the pharmaceutical composition according to the present invention, but specific examples thereof may comprise, but are not limited to, aging-related macular degeneration, exudative aging-related macular degeneration, choroidal neovascularization, pathological myopia, diabetic retinopathy, macular edema, retinal vein occlusion, premature retinopathy, or neovascular glaucoma, but the present invention is not limited thereto.

The term "prevention" as used herein means all actions that inhibit or delay the onset of cancer or angiogenesis-related diseases via administration of the pharmaceutical composition according to the present invention.

The term "treatment" as used herein means all actions that improve or beneficially change the symptoms of cancer or angiogenesis-related diseases via administration of the pharmaceutical composition according to the present invention.

The pharmaceutical composition according to the present invention may further comprise a pharmaceutically acceptable carrier. The pharmaceutically acceptable carrier may be a binder, a lubricant, a disintegrant, an excipient, a solubilizing agent, a dispersant, a stabilizer, a suspension agent, a pigment, a flavoring, or the like in the case of oral administration, may be used in combination with a buffer, a preservative, an analgesic agent, a solubilizer, an isotonic agent, a stabilizer, or the like in the case of injections, and may be a base, an excipient, a lubricant, a preservative, or the like in the case of topical administration. Preparations of the pharmaceutical composition of the present disclosure may be formulated in a variety of ways by mixing with the above-described pharmaceutically acceptable carrier(s). For example, preparations for oral administration may be formulated in the form of tablets, troches, capsules, elixirs, suspensions, syrups, wafers, or the like, and preparations for injection may be formulated in unit-dosage ampoules or in multiple-dosage form. In addition, the composition may typically include a surfactant that facilitates movement across a membrane. Such surfactants are those derived from steroids, cationic lipids such as N-[1-(2,3-dioleoyl)propyl-N,N,N-trimethylammoniumchloride(DOTMA) and the like, or various compounds such as cholesterol hemisuccinate, phosphatidyl glycerol, and the like.

The composition according to the present invention, which comprises the conjugate, may be administered in a pharmaceutically effective amount to treat cancer cells, metastasis thereof, or angiogenesis-related diseases, or to inhibit cancer growth. The pharmaceutically effective amount may vary depending on various factors such as the type of cancer, the type of angiogenesis-related disease, the age and body weight of the patient, the characteristics and severity of symptoms, the current treatment option, the number of treatments, administration form and route, and the like, and may be easily determined by those of ordinary skill in the art. The composition of the present invention may be simultaneously or sequentially administered in combination with pharmacological or physiological ingredients, may be administered in combination with additional conventional therapeutic agents, and may be administered sequentially or simultaneously with conventional therapeutic agents. Such administration may be a single or multiple administration. It is important to administer the composition in the minimum amount that enables achievement of the maximum effects without side effects in consideration of all of the above-described factors, and this may be easily determined by one of ordinary skill in the art.

Another embodiment of the present invention provides a method of preventing or treating cancer or angiogenesis-related disease comprising administering to a subject the conjugate or the pharmaceutical composition. Specifically, the method of preventing or treating cancer or angiogenesis-related disease according to the present invention may comprise administering the conjugate or the pharmaceutical composition to a subject other than a human, but the present invention is not limited thereto.

Here, the definitions of the terms "conjugate", "cancer", and "angiogenesis-related disease" are the same as those given above.

The term "subject" as used herein refers to all animals such as mice, rats, livestock, and the like, including humans who are in a state in which cancer or angiogenesis-related disease can be alleviated, suppressed, or treated by administration of the pharmaceutical composition according to the present invention; or who have or are at risk of contracting cancer or angiogenesis-related disease.

The term "administration" as used herein means introducing a predetermined substance into a subject using an appropriate method, and the pharmaceutical composition of the present invention may be administered via any general route as long as it allows the composition to reach the target tissue. The administration route may include, but is not limited to, intraperitoneal administration, intravenous administration, intramuscular administration, subcutaneous administration, intradermal administration, oral administration, topical administration, intranasal administration, intrapulmonary administration, and rectal administration. However, for oral administration, an oral composition may be formulated by coating the active ingredient, or may be formulated so as to protect the active ingredient from degradation in the stomach, since proteins are digested. In addition, the pharmaceutical composition may be administered by a device capable of transferring an active material to a target cell.

### [Mode for Invention]

Hereinafter, the present invention will be described in further detail with reference to the following examples. However, these examples are provided for illustrative purposes only and are not intended to limit the scope of the present invention.

### Example 1: Cell Lines and Cell Culture

Freestyle 293F cells (R790-07, Gibco®), A431 cells (human cervix epidermoid carcinoma, #21555, Korean Cell Line Bank), SKBR3 cells (human breast adenocarcinoma, #30030, Korean Cell Line Bank), SKOV3 cells (human ovarian adenocarcinoma, #30077, ATCC), and human umbilical vein endothelial cells (HUVECs, CC-2519, Lonza) were authenticated according to ATCC guidelines and used within 6 months of receipt. Freestyle 293F cells (R790-07, Gibco®) were maintained in suspension culture in Freestyle293F medium (12338018, Gibco®) at 37 °C and 8% CO₂ with 125 rpm agitation. A431 cells were cultured in DMEM (LM001-05, Welgene) supplemented with 10% heat-inactivated FBS (S001-01, Welgene) and 100 µg/ml of penicillin/streptomycin, SKBR3 cells and SKOV3 cells were cultured in RPMI1640 (LM011-05, Welgene) supplemented with 10% heat-inactivated FBS (S001-01, Welgene) and 100 µg/ml of penicillin/streptomycin, and HUVECs were cultured in EBM-2 (CC-3156, Lonza) supplemented with EGM-2 (CC-3162, Lonza) and penicillin/streptomycin on gelatin (G9391, Sigma-Aldrich; 2% in PBS) precoated plates. All cells were grown at 37 °C in 5% CO₂.

### Example 2: Antibodies

Antibodies used in the present invention are shown in Table 1 below.

**[Table 1]**

| Antibody name | Catalog No. | Manufacturer |
|---|---|---|
| Primary antibody | | |
| Rabbit anti-EGFR (WB) | CST-2232 | Cell Signaling |
| Rabbit anti-EGFR (IP) | Ab52984 | Abcam |
| Rabbit anti-phospho-EGFR (WB) | CST-2234 | Cell Signaling |
| Rabbit anti-phospho-EGFR (IP) | CST-3733 | Cell Signaling |
| Rabbit anti-Her2 (WB) | CST-2242 | Cell Signaling |
| Rabbit anti-phospho-Her2 (WB) | CST-2243 | Cell Signaling |
| Rabbit anti-VEGFR2 | CST-9698 | Cell Signaling |
| Rabbit anti-phospho-VEGFR2 | CST-2478 | Cell Signaling |
| Rabbit anti-AKT | CST-9272 | Cell Signaling |
| Rabbit anti-phospho-AKT | CST-4060 | Cell Signaling |
| Rabbit anti-ERK1/2 | CST-4695 | Cell Signaling |
| Rabbit anti-phospho-ERK1/2 | CST-9101 | Cell Signaling |
| Mouse anti-β-actin | sc-47778 | Santa-Cruz |
| Hamster anti-CD31 | MAB1398Z | Millipore |
| Secondary antibody | | |
| FITC-conjugated anti-hamster IgG | 127-095-009 | Jackson ImmunoResearch |
| Cy3-conjugated anti-rabbit IgG | 111-165-144 | Jackson ImmunoResearch |
| Alexa 488-conjugated anti-human IgG | A-11013 | Thermo Scientific |
| HRP-conjugated anti-rabbit IgG | sc-2004 | Santa-Cruz |
| HRP-conjugated anti-mouse IgG | sc-2005 | Santa-Cruz |

### Example 3: Expression and Purification of Recombinant Proteins

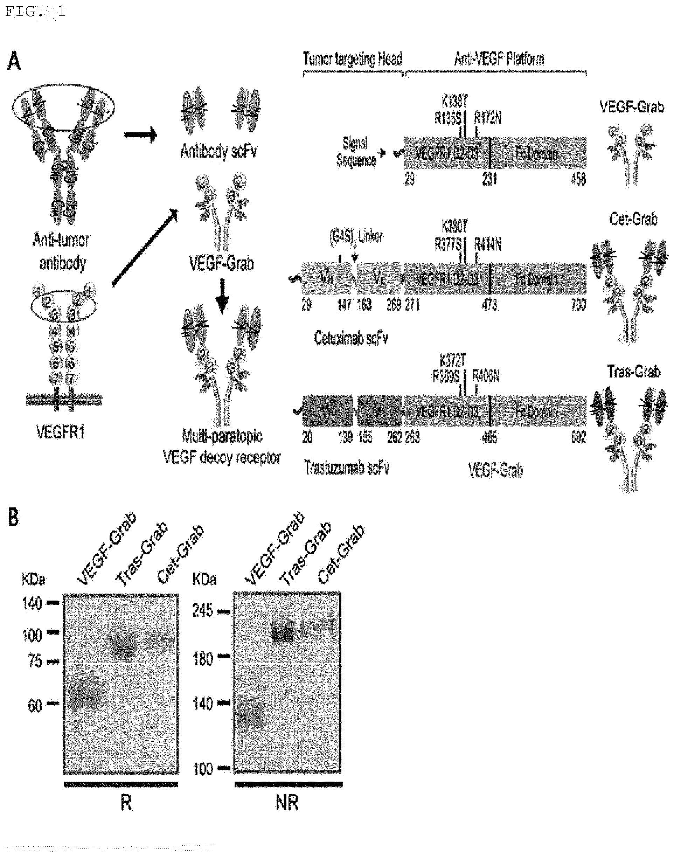

Genes encoding cetuximab or trastuzumab single chain variable fragment (scFv), in which the variable regions of cetuximab or trastuzumab's heavy and light chains were connected by a (G4S)3 linker (Ahmad ZA, Clin Dev Immunol. 2012, 2012: 980250.), were linked to the N-terminus of VEGF-Grab (Lee JE, Mol Cancer Ther., 2015, 14: 470-9) (see FIG. 1A). Vectors containing VEGF-Grab, scFv-Cetuximab-VEGF-Grab (Cet-Grab), and scFv-Trastuzumab-VEGF-Grab (Tras-Grab) were transfected into Freestyle293F cells using polyethyleneamine (765090, Sigma-Aldrich). The transfected cells were cultured for 3 days together with 5mM sodium butyrate (303410, Sigma-Aldrich), and then centrifuged using a centrifuge to separate only a supernatant. The supernatant containing VEGF-Grab, Cet-Grab, or Tras-Grab was purified using Protein A Sepharose (GE Healthcare Life Sciences). VEGF-Grab, Cet-Grab, or Tras-Grab was eluted with 200 mM glycine, pH 2.7, and then neutralized immediately with 1 M Tris-HCl (pH 8.0), dialyzed against PBS, and stored.

### Example 4: Binding Affinity Analysis

The binding affinities of a multi-paratopic-VEGF decoy receptor (Cet-Grab or Tras-Grab) to the EGFR family extracellular domain (EGFR for Cet-Grab; and HER2 for Tras-Grab), VEGF-A, or PlGF were analyzed through biolayer light interferometry using a BLITZ system (ForteBio, Pall Life Sciences). Biotinylated EGFR family ECD (EGFR or HER2), VEGF-A, or PlGF was bound to a streptavidin (SA) biosensor (1805020, ForteBio) previously hydrated for 2 minutes, followed by washing with PBS for 2 minutes to remove any unbound protein. Subsequently, each resulting product was allowed to react with 4 µl of VEGF-Grab, Cet-Grab, cetuximab, Tras-Grab, or trastuzumab (25-50 nM) and the association rate (kon) was measured at intervals of 2 minutes. Thereafter, to measure the dissociation rate (koff), each reaction product was allowed to react in a PBS buffer for 2 minutes. The final dissociation constant was calculated as a ratio of koff/kon. Sensorgrams were analyzed with the global fitting function using a 1:1 binding model (grouped by color and Rmax).

To analyze simultaneous binding to two targets, biotinylated VEGF family (VEGF-A or PlGF) was loaded onto SA biosensors for 90 seconds, and the VEGF pre-loaded biosensors were allowed to react with 4 µl of 100 nM multi-paratopic-VEGF decoy receptor (Cet-Grab or Tras-Grab) for 90 seconds. Subsequently, to measure the association rate (kon), the reaction product was allowed to react with 25-50 nM EGFR family ECD (EGFR for Cet-Grab; and HER2 for Tras-Grab) for 120 seconds. Thereafter, to measure the dissociation rate (koff), the reaction product was washed with PBS at intervals of 2 minutes.

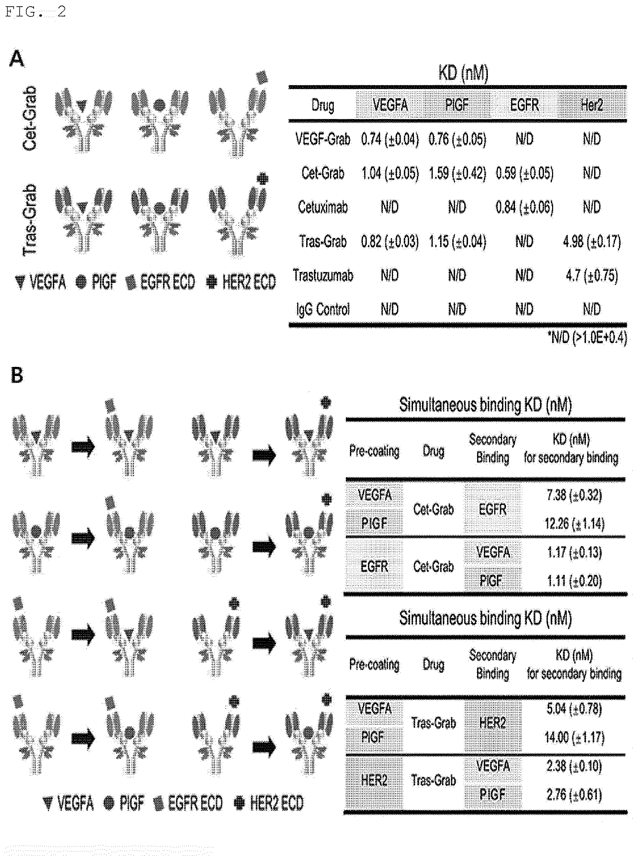

As illustrated in FIG. 2, pre-binding analysis of the EGFR family (EGFR for Cet-Grab; and HER2 for Tras-Grab) was performed using the same method as described above or in the reverse order.

### Example 5: Drug localization Analysis at Cellular Level

EGFR+ A431 cells incubated with 50 nM Cet-Grab, cetuximab, or VEGF-Grab (negative control) at 37 °C for 6 hours, and HER+ SKBR3 cells incubated with 50 nM Tras-Grab, trastuzumab, or VEGF-Grab at 37 °C for 6 hours were washed with PBS 3 times, fixed in 4% PFA (P2031, biosesang) for 20 minutes, and then permeabilized with a 0.5% Tween-20 in PBS solution at room temperature. Subsequently, to visualize the location of these proteins, the cells were stained using an Alexa-488 conjugated anti-human IgG antibody and then counterstained with DAPI. The stained cells were analyzed using a Carl Zeiss LSM780 confocal microscope, and fluorescence signals (Alexa-488 to DAPI) were quantified using Image J software.

### Example 6: Cell Viability Assays

Cancer cell lines (A431 cells and SKBR3 cells) were seeded onto 96-well plates (100 µl, 2,500 cells/well), and after 24 hours, the A431 cells were incubated for 48 hours with 1/2-fold serial dilutions of a maximum of 1 µM cetuximab or Cet-Grab, and the SKBR3 cells were incubated for 48 hours with 1/2-fold serial dilutions of a maximum of 1 µM trastuzumab or Tras-Grab. Thereafter, 10 µl of an Ez-cytox solution (EZ-3000, DAEILLAB) was added to each well, and then the absorbance at 450 nm was measured. The measurement values were analyzed using GraphPad PRISM 5 software, and then IC₅₀ was calculated.

### Example 7: Analysis of Inhibition of EGFR/HER2/VEGFR2 Signaling by Cet-Grab or Tras-Grab

For EGFR family (EGFR and HER2) signaling analysis, cancer cell lines (A431 cells or SKBR3 cells) were treated with 25 nM cetuximab or Cet-Grab (A431 cells) or with 25 nM trastuzumab or Tras-Grab (SKBR3 cells) for 48 hours. For VEGFR2 signaling analysis, HUVECs were treated with 25 nM of Cet-Grab, Tras-Grab, or VEGF-Grab for 15 minutes followed by treatment with 1 nM VEGF-A for 10 minutes. Proteins were isolated from the cells treated with each drug, using an RIPA solution (BRI-9001 T&I) containing a phosphatase inhibitor (56-25-7, Roche). 30 µg of proteins were separated using 10% (EGFR, HER2, VEGFR2, p-EGFR, p-HER2, p-VEGFR2, β-actin) or 12% (ERK, p-ERK) SDS-PAGE gels, followed by transfer to nitrocellulose membranes and western blotting with suitable antibodies (Table 1) (Lee JE, Mol Cancer Ther. 2015, 14: 470-9). A human IgG Fc domain was used as a negative control.

### Example 8: Colony Formation Analysis

EGFR+ A431 cells were cultured in a 5% CO₂ incubator at 37 °C for 21 days in the presence of cetuximab, Cet-Grab, or IgG Fc domain (negative control). HER2+ SKOV3 cells were cultured similarly as described above in the presence of trastuzumab, Tras-Grab, or IgG Fc domain (negative control). The cultured plates were washed with PBS, followed by fixation with 4% PFA and staining with a 0.05% crystal violet solution (C0775, Sigma-Aldrich) for 20 minutes at room temperature. Thereafter, colonies with a size of 1 mm or greater were quantified and analyzed.

### Example 9: Endothelial Cell Migration Assays

HUVECs were cultured using µ-dishes (Cat # 81176, Ibidi) until they became confluent. Subsequently, the inserts were removed and, the gaps were generated (Lee JE, Mol Cancer Ther., 2015, 14: 470-9). The cultures were incubated in EBM-2 medium (Lonza) containing 1 nM VEGF-A and 25 nM of each indicated proteins (VEGF-Grab, Cet-Grab, or Tras-Grab) for 24 hours, and migrated cells within the gaps were monitored.

### Example 10: Tube Formation Assay

HUVECs were dispensed into Matrigel-coated-96-well plates (354230, Corning) at a density of 6,000 cells/well, and then treated with VEGF-Grab, Cet-Grab, or Tras-Grab (2 µg/ml). After 10 minutes, 1 nM hVEGF-A was added and tube formation was monitored after 6 hours.

### Example 11: Mouse Xenograft Model

Athymic female nude mice, aged 4 weeks, were purchased from Nara Biotech (Seoul, Korea). 3 X 10⁶ A431 cells or 5 X 10⁶ SKOV3 cells were subcutaneously injected into the right dorsal shoulder of each mouse, and once tumors reached ∼50 mm³ in volume, PBS (control) or 10 mg/kg of VEGF-Grab, Cet-Grab (to EGFR+ A431 xenografts mouse model), or Tras-Grab (to HER2+ SKOV3 xenografts mouse model) was intraperitoneally administered to each mouse twice a week for 3 weeks (n=5). Tumor volume was calculated as length x width x height x 0.5. After administration and measurement were completed, each mouse was anesthetized, and then blood was collected therefrom and tumors were extracted therefrom for further analysis. This experiment was conducted after approved by the Institutional Animal Care and Use Committee at Korea Research Institute of Bioscience and Biotechnology (Approval No.: KRIBB-AEC- 16001).

### Example 12: Histological Analysis of Tumor Tissues

The extracted tumor tissues were fixed in 4% paraformaldehyde at 4 °C overnight, incubated in a 30% sucrose (S7902, Sigma-Aldrich, PBS) solution, and then frozen using an O.C.T. solution (4583, Tissue-Tek®). The frozen tissues were cryo-sectioned (40 µm, Leica), blocked with 5% normal serums, stained with a solution containing the designated antibodies, and then counterstained with DAPI. The stained tissues were analyzed by confocal microscope (Carl Zeiss LSM780), and fluorescence intensity was quantified and normalized to DAPI using ImageJ software.

### Example 13: In Vivo Drug Distribution Analysis (IVIS Imaging)

Cet-Grab, Tras-Grab, and VEGF-Grab were labeled with Cy5.5 using Cy5.5 Fast Conjugation Kit (ab195226, Abcam) according to the manufacturer's instructions, and separated from remaining free dye by Bio-Gel p6 DG gel filtration chromatography (Bio-Rad). Cy5.5-conjugated Cet-Grab, Tras-Grab or VEGF-Grab (10 mg/kg) was intraperitoneally administered to athymic nude mice into which A431 tumors (VEGF-Grab or Cet-Grab) or SKOV3 tumors (VEGF-Grab or Tras-Grab) grown to a size of about 100 mm³ were transplanted. 24 hours after administration, mice were anesthetized with isoflurane, and imaged with Cy5.5 channel using IVIS-200 (Xenogen). After 12 hours, mice were euthanized, tumors and major organs including the liver, kidneys, heart, and spleen were extracted, and the extracted tumors and organs were imaged. Resected tumors were fixed in PFA for further histologic analysis.

### Example 14: Statistical Analysis

All data are shown as mean ± SEM of at least three independent experiments, and statistical significance between groups were compared by a two-tailed student's *t*-test (* P <0.05; ** P <0.01; *** P <0.001).

### Example 15: Construction of Recombinant DNA

To generate Cetuximab-VEGF-Grab and Trastuzumab-VEGF-Grab, sequences encoding a single chain variable fragment of cetuximab (heavy chain-(G₄S)₃ linker-light chain) and trastuzumab were synthesized (Bioneer), amplified by PCR, and then cloned into the EcoRI/NotI site of the above-described vector pCMV-dhfr (Lee JE, Mol Cancer Ther. 2015, 14:470-9., Goldstein.). A sequence encoding the extracellular domain of human EGFR (25L-645S, NM005228.4) was amplified by PCR, and then cloned into the BamHI/XbaI site of a modified pCMV-dhfr vector containing a thrombin cleavage site and protein A tag.

### Example 16: Antibody-dependent Cellular Cytotoxicity (ADCC) Reporter Analysis

ADCC reporter analysis was performed on A431 cells according to the manufacturer's instructions (G7010, Promega). Briefly, A431 cells (7 X 10⁵ in ADCC assay buffer/well) were plated onto a 96-well plate. After 24 hours, each well was treated with VEGF-Grab, Cet-Grab, and cetuximab (maximum 1 µg/ml, 1/3-fold dilution for Cet-Grab and cetuximab, and 1/9-fold dilution for VEGF-Grab). Effector cells (5 X 10⁶, 25 µl) were added to each well in an E:T ratio of 7:1. Subsequently, the cells were incubated at 37 °C for 6 hours, and a luciferase assay reagent (75 µl) was added to each well to measure luminescence. The measurement and analysis of luminescence were performed using GraphPad prism5.

### Experimental Example 1: Design of Multi-paratopic VEGF Decoy Receptor and Confirmation of Properties thereof

To confirm whether the fusion of VEGF Grab with an anti-EGFR therapeutic antibody enhances tumor targeting activity of VEGF-Grab, the present inventors designed novel multi-paratopic VEGF decoy receptors called Cetuximab-VEGF-Grab (SEQ ID NO: 11) and Trastuzumab-VEGF-Grab (SEQ ID NO: 24) (hereinafter referred to as Cet-Grab and Tras-Grab) produced by fusing the single chain variable fragment (scFv) (SEQ ID NO: 9) of cetuximab (anti-EGFR antibody) or scFv (SEQ ID NO: 20) of trastuzumab (anti-HER2 antibody) with VEGF-Grab (SEQ ID NO: 22).

First, a sequence encoding the scFv domain of cetuximab or trastuzumab (V_{H}-(G₄S)₃ linker-V_{L}) (Ahmad ZA, Clin Dev Immunol. 2012, 2012: 980250) was synthesized and fused to the N-terminus of VEGF-Grab in pcDNA3.1 vector (Lee JE, Mol Cancer Ther., 2015, 14 : 470-9) through recombination techniques (see FIG. 1A). Cet-Grab and Tras-Grab were produced using Freestyle293F cells, purified by affinity chromatography, and analyzed by SDS-PAGE under reducing conditions (R) and nonreducing conditions (NR), respectively (see FIG. 1B). As a result of SDS-PAGE analysis, the molecular weights (MWs) of the purified proteins were slightly higher than the calculated values (VEGF-Grab, 97.2 kDa; cetuximab, 145.8 kDa; Cet-Grab, 149.2 kDa; Tras-Grab, 149 kDa, in a dimeric state) due to their glycosylation.

### Experimental Example 2: Confirmation of Multi-specificity of Multi-paratopic VEGF Decoy Receptors to VEGF and EGFR Family

The binding affinities of multi-paratopic VEGF decoy receptors (Cet-Grab or Tras-Grab) to respective target proteins, VEGF family (human VEGF-A and PlGF) and EGFR family (human EGFR for Cet-Grab; and HER2 for Tras-Grab) were analyzed by biolayer light interferometry (BLI) using a Blitz (ForteBio) system.

As a result, the binding affinities (K_{D}) of VEGF-A and PlGF to Cet-Grab were 1.04 nM and 1.59 nM, respectively, and the binding affinities (K_{D}) of VEGF-A and PlGF to Tras-Grab were 0. 82 nM and 1.15 nM, respectively, which are comparable with those to VEGF-Grab (VEGF-A, 0.74nM; PlGF, 0.76 nM). Analysis results for the binding affinity to outer-paratopes of Cet-Grab or Tras-Grab showed that the binding affinity of an EGFR extracellular domain (ECD) to Cet-Grab was 0.59 nM, and the binding affinity of HER2 ECD to Tras-Grab was 4.98 nM. It was also confirmed that VEGF-Grab did not interact with the EGFR family (EGFR ECD and HER2 ECD) and the binding affinity of parental antibodies to their respective targets (the binding affinity of cetuximab to EGFR ECD, 0.84nM; and the binding affinity of trastuzumab to HER2, 4.7 nM) did not differ significantly from those of Cet-Grab and Tras-Grab (see FIG. 2A).

The above results suggest that the fusion of cetuximab (anti-EGFR antibody) scFv or trastuzumab (anti-HER2 antibody) scFv to VEGF-Grab does not influence their binding properties toward respective target proteins, VEGF family and EGFR family.

Next, concurrent binding of the above targets to multi-paratopic VEGF decoy receptors was examined. First, the binding affinity of outer-paratopes of Cet-Grab and Tras-Grab to EGFR ECD and HER2 ECD, respectively, was assessed when VEGF-A or PlGF was allowed to pre-bind to inner-paratopes thereof.

As a result, the second binding affinities of Cet-Grab and Tras-Grab to the EGFR family ECD were slightly weakened (the second binding affinity of Cet-Grab/VEGF-A to EGFR ECD, 7.38 nM; the second binding affinity of Cet-Grab/PlGF to EGFR ECD, 12.26 nM; the second binding affinity of Tras-Grab/VEGF-A to HER2 ECD, 5.04 nM; and the second binding affinity of Tras-Grab/PlGF to HER2 ECD, 14.00 nM), but it was sufficient to maintain their concurrent binding to the VEGF family and the EGFR family ECD. Similarly, pre-binding of EGFR ECD or HER2 ECD to the outer-paratope of Cet-Grab or Tras-Grab did not affect the binding affinity of their inner-paratopes to VEGF-A or PlGF (the binding affinity of Cet-Grab/EGFR ECD to VEGF-A, 1.17 nM; the binding affinity of PlGF to Cet-Grab/EGFR ECD, 1.11 nM; the binding affinity of VEGF-A to Tras-Grab/HER2 ECD, 2.38 nM; and the binding affinity of Tras-Grab/HER2 ECD to PlGF, 2.76 nM) (see FIG. 2B).

From the above results, it indicates that Cet-Grab and Tras-Grab have multi-specificity to respective target proteins with a comparable binding affinity as parental VEGF-Grab and anti-EGFR therapeutic antibodies (cetuximab and trastuzumab), and are able to simultaneously bind to their target proteins, i.e., the VEGF family and the EGFR family.

### Experimental Example 3: Confirmation of Inhibition of HUVEC Migration and Tube Formation through Suppression of VEGF Signaling Pathways by Multi-paratopic VEGF Decoy Receptors

To investigate whether multi-paratopic VEGF decoy receptors are able to inhibit the activation of VEGF-A-induced HUVECs by blocking VEGF-A, first, VEGF-A-induced VEGFR2 signaling in HUVECs was examined.

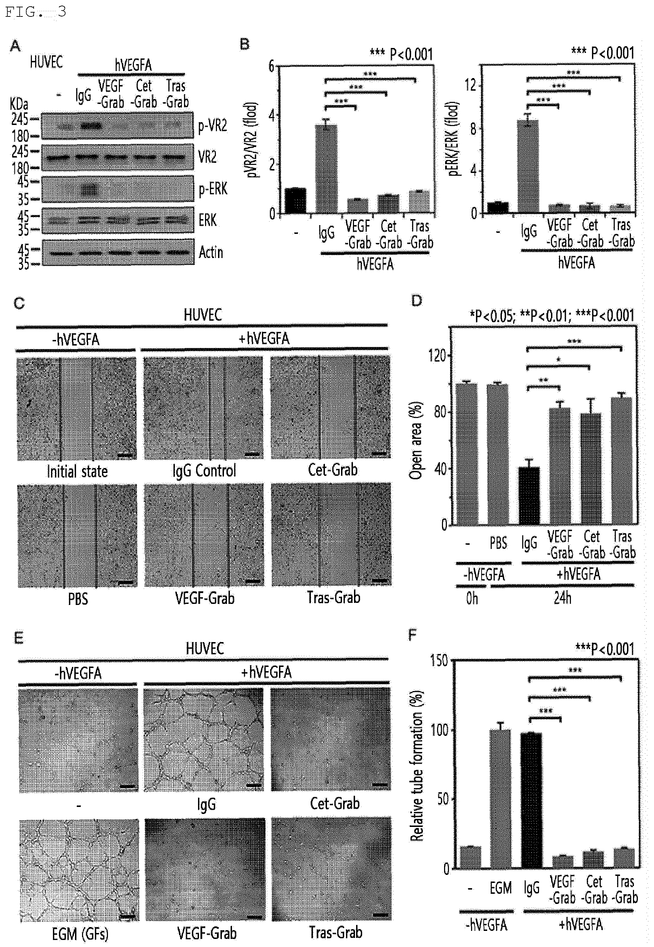

As a result, similar to VEGF-Grab, both Cet-Grab and Tras-Grab attenuated VEGF-A-induced phosphorylation of VEGFR2 and its downstream ERK signaling (see FIGS. 3A and 3B).

In addition, since VEGF-A is known to promote proliferation, migration, and survival of endothelial cells by activating VEGFR2, it was examined whether migration and tube formation of HUVECs inducible by VEGF-A were inhibited by Cet-Grab and Tras-Grab.

As a result, consistent with the results of VEGFR2 signaling inhibition, Cet-Grab and Tras-Grab strongly suppressed VEGF-A-induced migration (see FIGS. 3C and 3D) and tube formation (see FIGS. 3E and 3F) of HUVECs without any significant differences.

Taken together, these results demonstrate that both Cet-Grab and Tras-Grab have similar binding affinities to VEGF-A, and thus have anti-VEGF activity similar to that of VEGF-Grab, and accordingly are able to effectively inhibit the activation of HUVECs.

### Experimental Example 4: Confirmation of Blocking of EGFR Pathway-mediated Cell Proliferative Signaling by Multi-paratopic VEGF Decoy Receptors

To evaluate the functional properties of scFv in Cet-Grab and Tras-Grab, it was examined whether these can bind onto EGFR-expressing tumors using A431 and SKBR3 cancer cells, which express high levels of EGFR and HER2, respectively.

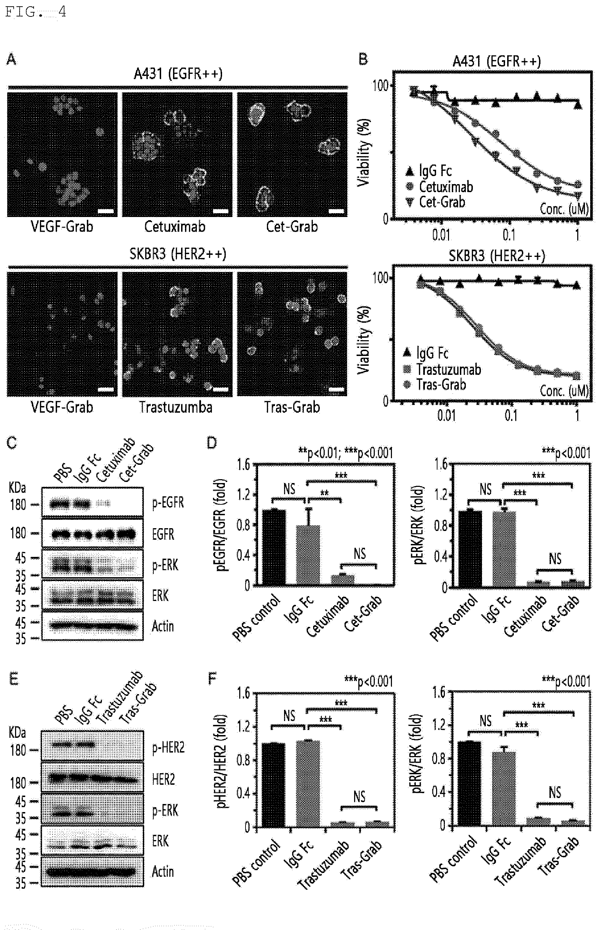

As a result of immunofluorescence staining, it was confirmed that Cet-grab and Tras-Grab stably bound to EGFR+ A431 and HER2+ SKBR3 cancer cells, respectively, whereas VEGF-Grab was unable to bind thereto (see FIG. 4A).

In addition, since it is known that binding of cetuximab and trastuzumab to cancer cells can effectively suppress tumor cell growth by preventing the binding of a ligand to EGFR or HER2 and blocking the auto-phosphorylation and activation of a receptor through the inhibition of receptor dimerization, and thus the anti-tumor activity of Cet-Grab and Tras-Grab was examined.

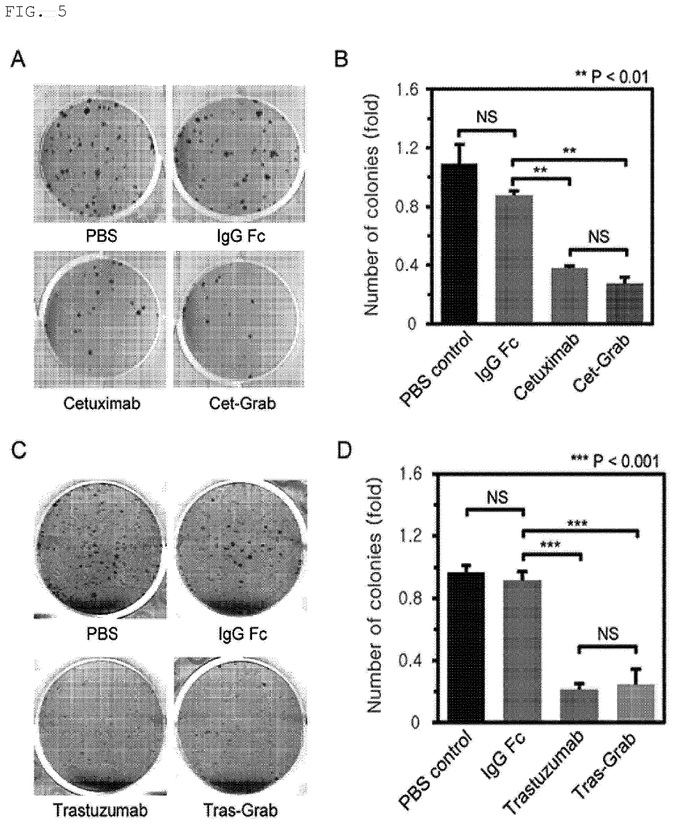

As a result of cell viability assays using A431 and SKBR3 cell lines, it was confirmed that Cet-Grab and Tras-Grab efficiently suppressed the proliferation of A431 cells and SKBR3 cells, respectively (FIG. 4B; IC₅₀ = 27.6 nM for Cet-Grab, IC₅₀ = 76.7 nM for cetuximab, IC₅₀ = 27.8 nM for Tras-Grab, IC₅₀ = 24.3 nM for trastuzumab). In addition, treatment with Cet-Grab significantly reduced phosphorylation levels of EGFR and its downstream ERK in A431 cells as cetuximab did (see FIGS. 4C and 4D), and similarly, Tras-Grab significantly reduced the HER2-mediated proliferation signaling in a SKBR3 cell line at a level similar to trastuzumab (see FIGS. 4E and 4F). Clonogenic assays also showed that the formation of colonies was dramatically attenuated in Cet-Grab- or Tras-Grab-treated cancer cells compared with PBS-treated cancer cells (see FIG. 5).

Taken together, it indicates that Cet-Grab and Tras-Grab have strong anti-EGFR activity and anti-HER2 activity, respectively, and thus are able to effectively suppress the proliferation and unlimited division of EGFR/HER2 overexpressing cancer cells.

### Experimental Example 5: Confirmation of Tumor Targeting Activity of Multi-paratopic VEGF Decoy Receptors in Xenograft Mouse Tumor Model

To assess tumor targeting of Cet-Grab and Tras-Grab, the distribution of Cet-Grab and Tras-Grab was directly monitored at *in vivo* xenograft mouse models. Specifically, EGFR+ A431 or HER2+ SKOV3 cancer cells were ectopically implanted into mice, and then when the tumor size reached ~ 100 mm³, Cy5.5-labeled Cet-Grab or Tras-Grab (10 mg/kg) was intraperitoneally injected to A431 and SKOV3 xenograft mice, respectively, and the *in vivo* distribution of Cet-Grab and Tras-Grab was monitored by analyzing Cy5.5 fluorescence signals. In addition, Cy5.5-labeled VEGF-Grab was used as a control in both A431 xenograft mice and SKOV3 xenograft mice.

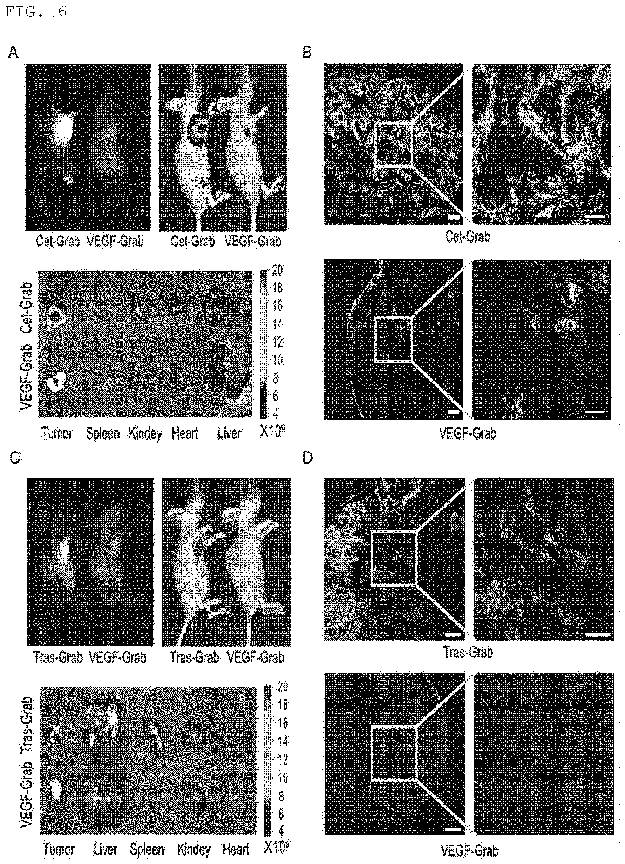

As a result of conducting the experiment, in balb-c/nude mice bearing EGFR+ A431 tumors, most Cy5.5-Cet-Grab was specifically localized at the tumor area (right dorsal shoulder), while Cy5.5-VEGF-Grab was distributed throughout the entire body (FIG. 6A, upper panel). Assessment of major organs of mice with tumors showed that Cet-Grab was accumulated at a high level in the tumors, but was barely present in other organs (see FIG. 6A, bottom panel). In addition, the results of immunofluorescence analysis of tumor sections showed that Cet-Grab was evenly distributed in both peri- and intra-tumoral areas, whereas VEGF-Grab was mostly localized to peri-tumoral areas (see FIG. 6C). Similar to the above results, most Cy5.5-Tras-Grab was specifically localized at HER2+ SKOV3 tumor area.

These results indicate that VEGF decoy receptors (Cet-Grab and Tras-Grab), in which scFv of cetuximab or trastuzumab was fused to VEGF-Grab, exhibit enhanced *in vivo* tumor targeting efficiency compared to parental VEGF-Grab (see FIGS. 6B and 6D).

### Experimental Example 6: Confirmation of Enhanced Anti-tumor Effect of Multi-paratopic VEGF Decoy Receptors in Xenograft Mouse Model

The anti-tumor effects of multi-paratopic VEGF decoy receptors *in vivo* were evaluated.

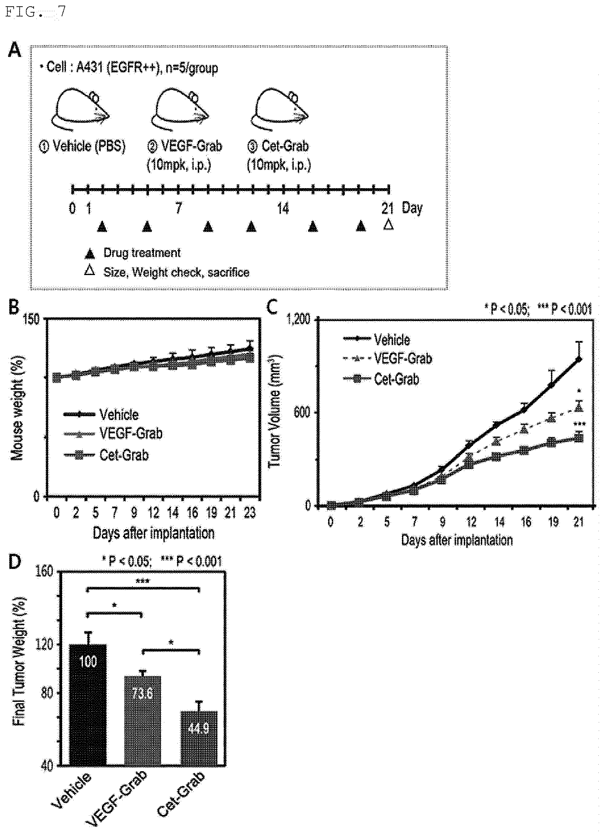

First, an EGFR+ A431 cell xenograft mouse model was treated with 10 mg/kg of Cet-Grab or VEGF-Grab twice a week for 3 weeks (see FIG. 7A), and as a result, Cet-Grab treatment did not affect mouse total weight (see FIG. 7B) and reduced tumor volume and weight by 57% and 55%, respectively, whereas VEGF-Grab treatment reduced tumor volume and weight by 25.2% and 26.4%, respectively (see FIGS. 7C and 7D).

According to the above results, to analyze the mechanism showing the greater anti-tumor effect of Cet-Grab than VEGF-Grab, EGFR signaling in isolated tumor tissue and changes in tumor vasculature were investigated.

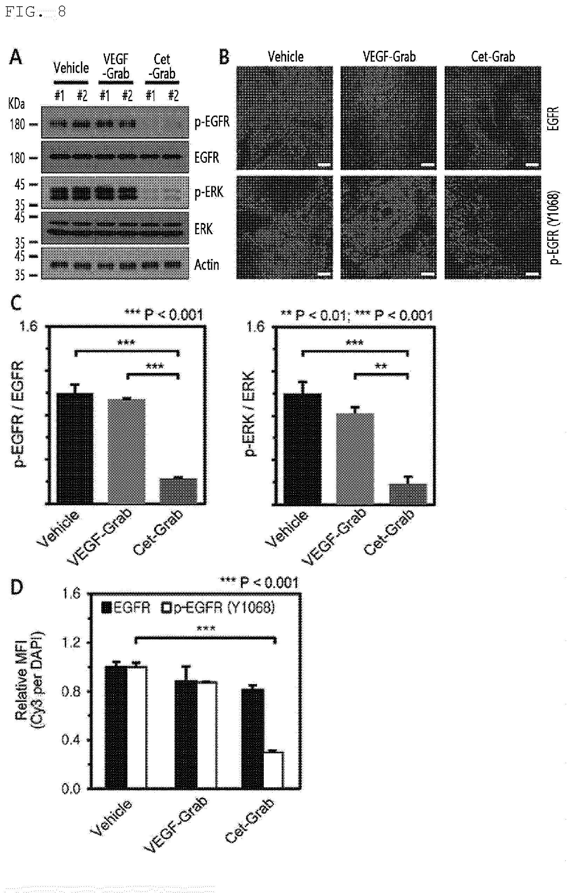

Consistent with the results of cell-based assays, from western blotting analysis and immunofluorescence staining results, it was confirmed that, EGFR phosphorylation in isolated tumor tissue was significantly decreased in Cet-Grab-treated mice compared to VEGF-Grab-treated mice although total EGFR level remained unchanged. Furthermore, the inactivation of EGFR was accompanied by markedly reduced ERK1/2 phosphorylation in Cet-Grab-treated mice compared to a vehicle-treated group. Although VEGF-Grab was able to attenuate the VEGF-mediated ERK phosphorylation in HUVECs, it did not influence on the EGFR phosphorylation and subsequent EGFR-mediated ERK phosphorylation in A431-derived tumor tissue due to its defective anti-EGFR activity (see FIG. 8).

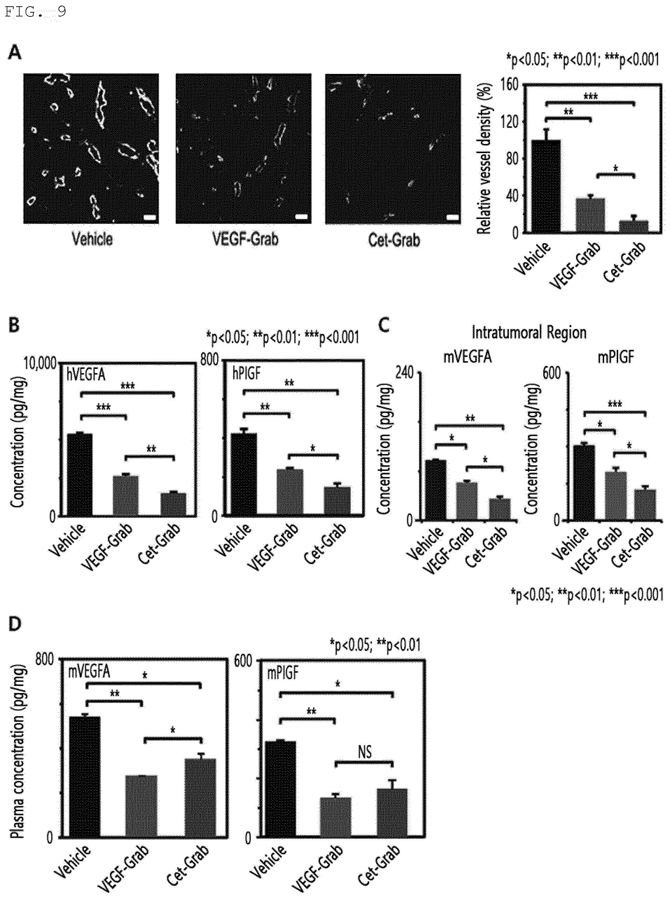

In addition, treatment with VEGF-Grab and Cet-Grab to A431 xenograft tumor models significantly reduced the number of blood vessels infiltrating tumors compared to a vehicle, and the thickness and continuity of tumor vessels in Cet-Grab group (87.2%) were attenuated more than that in VEGF-Grab group (63%) (see FIG. 9A). As a result of measuring levels of the VEGF-A and PlGF proteins that can be sequestered by VEGF-Grab and Cet-Grab in both serum and tumor tissue, despite VEGF-Grab having slightly higher affinity for VEGF and PlGF than Cet-Grab, the levels of human and mouse VEGF (VEGF-A and PlGF) at intra-tumoral area, which are secreted from human A431 cancer cells and other cells in tumor microenvironment respectively, were markedly lower in Cet-Grab-treated group than in VEGF-Grab-treated group (see FIGS. 9B and 9C). However, the plasma concentration of mVEGF was slightly higher in Cet-Grab-treated mice than in VEGF-Grab-treated mice (see FIG. 9D), which indicates that Cet-Grab was specifically localized at EGFR+ A431 tumor area, thus enabling targeted anti-VEGF activity, which can potentially reduce the side effect of systemic inhibition of VEGF signaling.

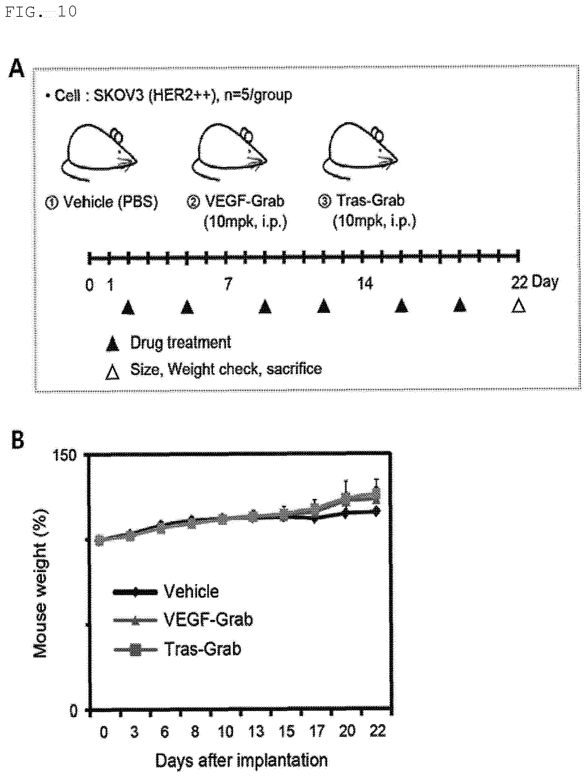

Similar to Cet-Grab, the treatment of Tras-Grab effectively suppressed HER2 phosphorylation and reduced the numbers of blood vessels infiltrating tumors in a HER2+ SKOV3 xenograft mouse model (see FIG. 10). The anti-tumor efficacy of Tras-Grab in the HER2+ SKOV3 xenograft mouse model was slightly enhanced compared to VEGF-Grab-treated group, but not as significant as that of Cet-Grab in an A431 xenograft model. This can be explained by the sustained ERK phosphorylation in a subset of HER2+ SKOV3 xenograft mouse model despite the effective suppression of HER2 phosphorylation *in vivo* by Tras-Grab treatment.

These results indicate that intrinsic or acquired resistance of HER2+ SKOV3 tumor cells may limit the effectiveness of Tras-Grab, which can be further improved by a combination of Tras-Grab and other agents that inhibit growth-promoting downstream signaling.

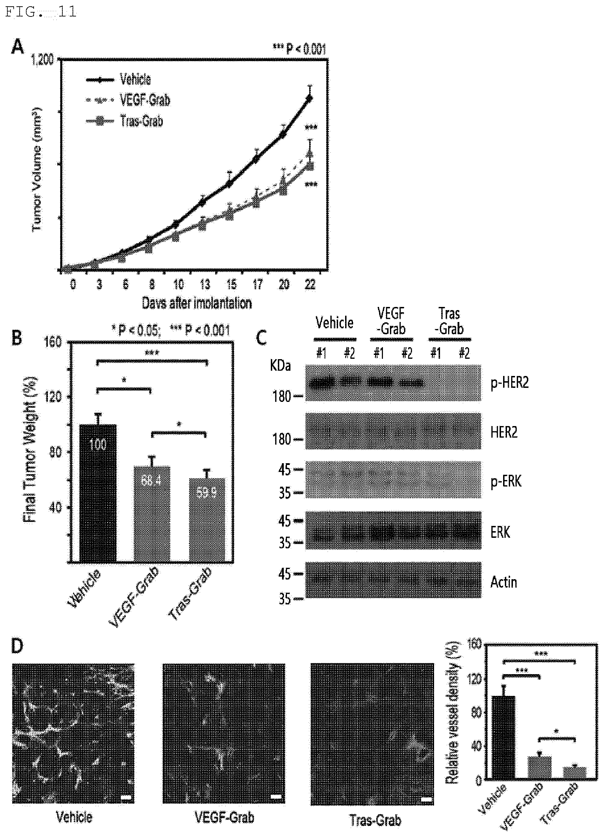

In addition, the anti-cancer efficacy of cetuximab is attributed to activation of the immune system, including antibody-dependent cellular cytotoxicity (ADCC) and complement-dependent cytotoxicity (CDC), and the ADCC and CDC are mediated mainly by the binding of FcγIII receptors on immune effector cells and C1q, respectively, to antigen-antibody complexes, particularly to the upper CH2 domain and the hinge between the CH1 and CH2 domains of the antibody. Since immunodeficient mice were used as a xenograft model of human cancer cells in the above experiment, it was impossible to directly evaluate the effect of Cet-Grab on ADCC *in vivo.* However, as a result of cell-based ADCC reporter analysis, Cet-Grab exhibited only marginal ADCC activity compared with cetuximab (see FIG. 11), probably due to the absence of the hinge sequence in Cet-Grab.

Taken together, multi-paratopic VEGF decoy receptors (Cet-Grab and Tras-Grab) exhibited the enhanced anti-tumor activity compared to VEGF-Grab at *in vivo* xenograft mouse tumor model, which may be attributed not only to its effective suppression of EGFR signaling but to its tumor-specific anti-angiogenic activity resulting from the specific localization of multi-paratopic VEGF decoy receptors at EGFR/HER2-overexpressing tumor tissue.

From the foregoing description of the present invention, it will be understood by those of ordinary skill in the art to which the present invention pertains that the present invention may be embodied in other particular forms without changing the technical spirit or essential characteristics thereof. Thus, the above-described examples and experimental examples should be construed as being provided for illustrative purposes only and not for purposes of limitation. The scope of the present invention should be construed that all changes or modified forms derived from the meanings and scope of the appended claims and equivalent concepts rather than the detailed description fall within the scope of the present invention.

## Claims

1. A conjugate of a fusion protein and a drug, wherein the fusion protein comprising a vascular endothelial growth factor receptor 1 (VEGFR1) domain 2, a VEGFR1 domain 3, and an antibody fragment.

2. The conjugate according to claim 1, wherein the antibody fragment comprises an Fc region of an antibody.

3. The conjugate according to claim 1, wherein the drug is an anti-cancer agent or a therapeutic agent for angiogenesis-related disease.

4. The conjugate according to claim 1, wherein the drug is an antibody capable of binding to epidermal growth factor receptor (EGFR) or human epidermal growth factor receptor type2 (HER2).

5. The conjugate according to claim 1, wherein the drug is cetuximab or trastuzumab.

6. The conjugate according to claim 1, wherein the drug has a form selected from the group consisting of scFv, dsFv, Fab, Fab', F(ab')₂, and nanobody, wherein the forms being antigen recognition sites.

7. A polynucleotide encoding the conjugate according to any one of claims 1 to 6.

8. An expression vector comprising the polynucleotide of claim 7.

9. A transformant comprising the expression vector of claim 8.

10. A method of producing a conjugate, comprising culturing the transformant of claim 9.

11. A pharmaceutical composition for preventing or treating cancer or angiogenesis-related disease, comprising the conjugate of claim 1.

12. The pharmaceutical composition according to claim 11, wherein the cancer is liver cancer, lung cancer, pancreatic cancer, non-small cell lung cancer, colon cancer, bone cancer, skin cancer, head or neck cancer, skin or intraocular melanoma, uterine cancer, ovarian cancer, rectal cancer, gastric cancer, anal muscle cancer, breast cancer, fallopian tube carcinoma, endometrial carcinoma, cervical carcinoma, vaginal carcinoma, vulva carcinoma, Hodgkin's disease, esophageal cancer, small intestine cancer, endocrine adenocarcinoma, thyroid cancer, parathyroid cancer, adrenal cancer, soft-tissue sarcoma, urethral cancer, penile cancer, prostate cancer, chronic or acute leukemia, lymphocytic lymphoma, bladder cancer, kidney or ureter cancer, renal cell carcinoma, renal pelvic carcinoma, central nervous system (CNS) tumor, primary central nervous system lymphoma, spinal cord tumor, brain stem glioma, or pituitary adenoma.

13. The pharmaceutical composition according to claim 11, wherein the angiogenesis-related disease is aging-related macular degeneration, exudative aging-related macular degeneration, choroidal neovascularization, pathological myopia, diabetic retinopathy, macular edema, retinal vein occlusion, premature retinopathy, or neovascular glaucoma.

14. A method of preventing or treating cancer or angiogenesis-related disease, comprising administering to a subject the conjugate of claim 1 or the pharmaceutical composition of claim 11.
